# EUROPEAN PATENT SPECIFICATION

(11) **EP 2 094 679 B1**
(45) Date of publication and mention of the grant of the patent: **25.01.2012**
(21) Application number: 07857390.4
(22) Date of filing: 11.12.2007
(51) Int. Cl.: C07D 317/12, C07D 317/24

(54) **PROCESS FOR PREPARING 3,5-DI-OMICRON-ACYL-2-FLUORO-2-C-METHYL-D-RIBONO-GAMMA-LACTONE**
VERFAHREN ZUR HERSTELLUNG VON 3,5-DI-OMIKRON-ACYL-2-FLUOR-2-C-METHYL-D-RIBONO-GAMMA-LACTON
PROCÉDÉ DE PRÉPARATION DE 3,5-DI-OMICRON-ACYL-2-FLUORO-2-C-MÉTHYL-D-RIBONO-GAMMA-LACTONE

(30) Priority: 18.12.2006 US 875617 P
(43) Date of publication of application: 02.09.2009
(73) Proprietor: F. Hoffmann-La Roche AG, 4070 Basel (CH)
(72) Inventor: CEDILOTE, Miall, Florence, South Carolina 29505 (US); CLEARY, Thomas P., Florence, South Carolina 29501 (US); IDING, Hans, 79618 Rheinfelden (DE); ZHANG, Pingsheng, Florence, South Carolina 29501 (US)
(74) Representative: Rauber, Beat
(86) International application number: PCT/EP2007/063703
(87) International publication number: WO 2008/074693

(56) References cited:
- WO-A-2006/012440

## Description

The present invention provides a novel process for the preparation of 3,5-di-O-acyl-2-fluoro-2-C-methyl-D-ribono-y-lactone derivatives of formula. 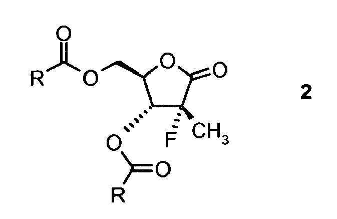 which is a key intermediate in the preparation of 1-(2-deoxy-2-fluoro-2-C-methyl-β-D-ribofuranosyl)cytosine 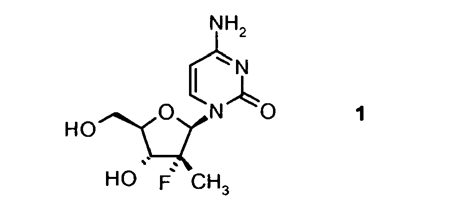 a compound that possesses potent and selective anti-hepatitis C virus activity (PCT Publication WO 2005/003147).

A number of synthetic routes for preparing intermediate 2 have been disclosed in PCT Publication WO 2006/012440, but these synthetic routes have the shortcomings of high manufacturing costs and technical difficulties for commercial scale manufacturing. The use of heavy load of asymmetric dihydroxlyation catalyst (AD-mix-β), fluorinating agent diethylaminosulfur trifluoride, and the Wittig reagent, are major cost drivers. The use of highly toxic reagents, such as AD-mix-β, highly reactive reagent such as diethylaminosulfur trifluoride, and chromatographic isolation of intermediates,contribute to scale up difficulties.

Object of the present invention was to find an alternative synthetic approach which does not suffer from the disadvantages known from the synthesis known in the art.

The object could be reached with the process of the present invention as outlined below.

The process for the preparation of a compound of the formula: 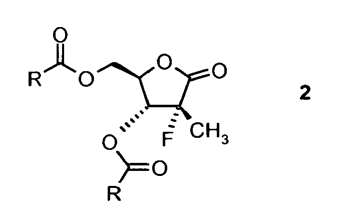 wherein R independently is C₁₋₆-alkyl, optionally substituted phenyl-C₁₋₆-alkyl or optionally substituted phenyl comprises one or more of the steps:
(a) reacting a compound of the formula 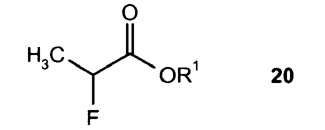 wherein R¹ is C₁₋₄-alkyl,
   with a base and a compound of formula 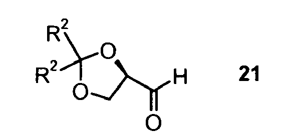 wherein R² is C₁₋₄-alkyl,
   to form a mixture of the compounds 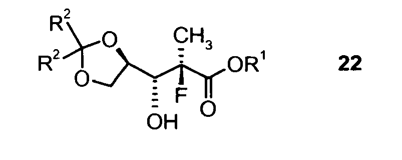 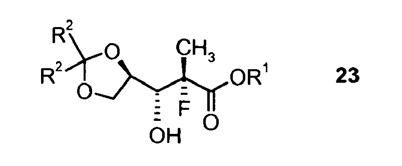 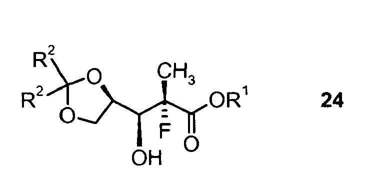 wherein R¹ and R² are as above;
b) reacting a mixture of the compounds 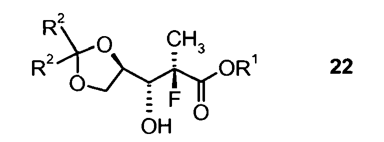 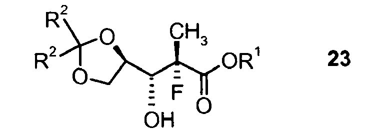 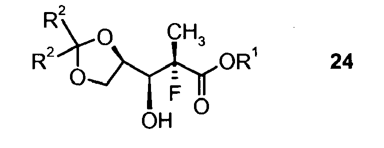 wherein R¹ and R² are as above,
   with an enzyme capable to hydrolyze the compound of formula 23 to the corresponding carboxylic acid and isolating a mixture of the compounds 22 and 24;
c) transforming a mixture of the compounds 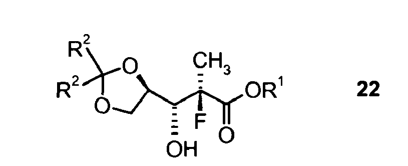 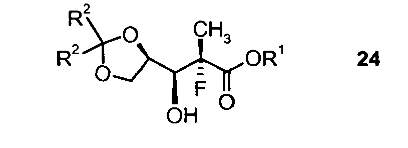 wherein R¹ and R² are as above, by acidic treatment into a mixture of the compounds 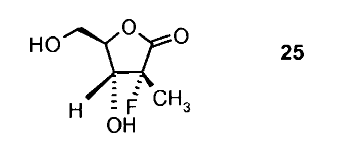 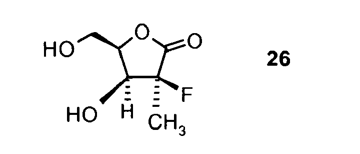
d) acylating a mixture of the compounds 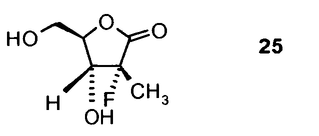 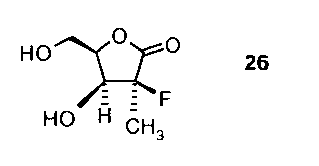 with an acyl halogenide of the formula RCOX wherein R is as above and X is a halogen or with an acyl anhydride RC(O)O(O)CR wherein R is as above in the presence of a base to form a mixture of the compounds 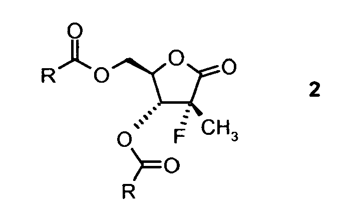 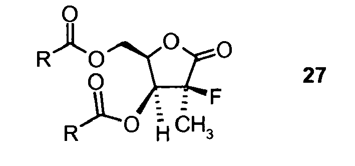 wherein R is as above;
e) recyrystallizing a mixture of the compounds 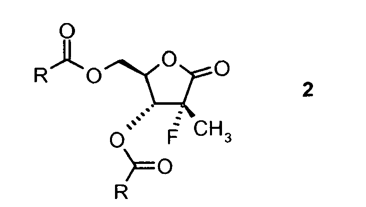 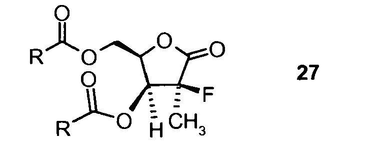 wherein R is as above in an organic solvent and isolating the compound of formula 2.

As used herein, the following terms have the meanings:

The term "phenyl-C₁₋₆-alkyl", as used herein refers to C₁₋₆-alkyl substituted with a phenyl group, preferably to benzyl.

The term "optionally substituted phenyl-C₁₋₆-alkyl" or "optionally substituted phenyl" refers to C₁₋₃-alkyl substituted phenyl-C₁₋₆-alkyl or C₁₋₃-alkyl substituted phenyl.

The term "C₁₋₄-alkyl" or "C₁₋₆-alkyl" refers to methyl, ethyl, n-propyl, iso-propyl, n-butyl, sec-butyl, t-butyl or in addition to pentyl and its isomers and hexyl and its isomers.

The term "halogen" refers to chloro, bromo, iodo and fluoro, and is preferably chloro.

In a preferred embodiment of the process of the present invention the substituents have the following meaning: R is phenyl, R₁ is methyl, ethyl, or n-butyl, preferably ethyl, R₂ is methyl or ethyl, preferably methyl and X is chloro.

### Step a)

Step a) requires reacting a compound of the formula 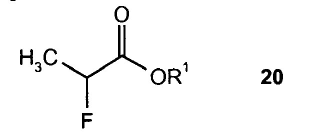 wherein R¹ is C₁₋₄-alkyl, with a base and a compound of formula 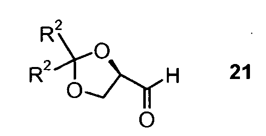 wherein R² is C₁₋₄-alkyl, to form a mixture of the compounds 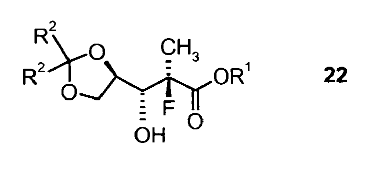 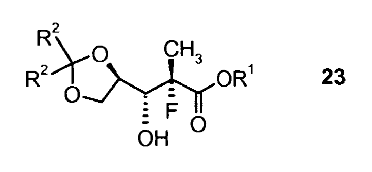 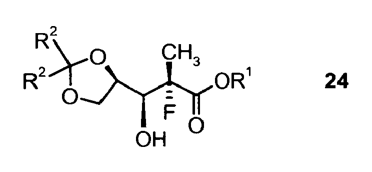 wherein R¹ and R² are as above.

The base used for the reaction step a) is a non nucleophilic base selected from the group consisting of lithium diisopropylamide (LDA), lithium 2, 2, 6, 6-tetramethylpiperidine (LTMP), and lithium hexamethyldisilazide (LHMDS).

The reaction in step (a) can be carried out in a suitable organic non-reactive solvent. Illustrative examples include tetrahydrofuran (THF), 2-Me-THF, toluene, diethyl ether, t-butyl methyl ether, and the like.

The reaction temperature is a rule maintained below -55°C. Isolation of the mixture of the compounds of formula 22, 23 and 24 can happen by adding an aqueous ammonium chloride solution to the reaction mixture and by extracting the aqueous phase with a suitable organic solvent such as with dichloromethane.

### Step b)

Step b) requires reacting a mixture of the compounds 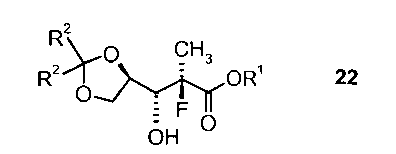 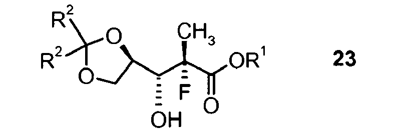 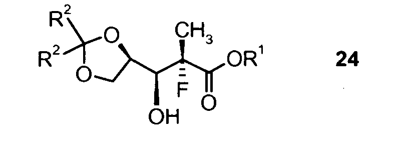 wherein R¹ and R² are as above,
with an enzyme capable to hydrolyze the compound of formula 23 to the corresponding carboxylic acid and isolating a mixture of the compounds 22 and 24.

In principle any enzyme may be employed which preferentially hydrolyzes compound 23 to the corresponding carboxylic acid in the presence of compounds 22 and 24.

Useful enzymes, which preferentially hydrolyze compound 23 in the presence of compound 22, include the lipase TOY from Pseudomonas aeruginosa (Toyobo); the esterase HLE from horse liver (Fluka), the esterase MME from Mucor miehei (Fluka); the esterase CLE from Candida lipolytica (Fluka); the lipase L1 from Burholderia cepacia (Roche); and the purified formulation of CALB which is the of lipase L2 from Roche.

*Candida Antarctica* lipase form B (CALB), Novozymes, is a preferred enzyme because of its high reactivity and stereoselectivity. Preferably, the selective hydrolysis of compound 23 is carried out with CALB at a temperature of about 20-45 °C in an aqueous buffer of about pH 7.0-7.5 that may contain ammonium sulfate, potassium sulfate, sodium sulfate, dimethylammonium phosphate, sodium acetate, sodium citrate, sodium phosphate, sorbitol, sucrose, glycine or other beneficial additives for the enzymatic activity.

The isolation of the mixture of compounds 22 and 24 from the alkaline aqueous medium may be carried out by way of extraction with a suitable organic solvent, preferably with dichloromethane.

### Step c)

Step c) requires transforming a mixture of the compounds 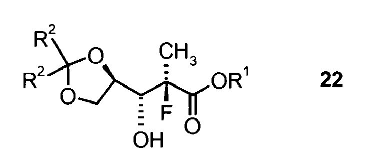 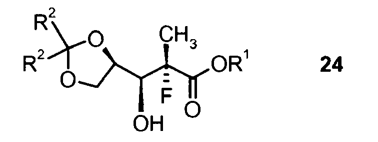 wherein R¹ and R² are as above, by acidic treatment into a mixture of the compounds

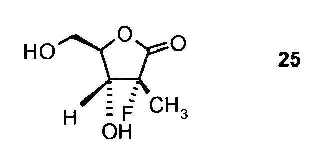

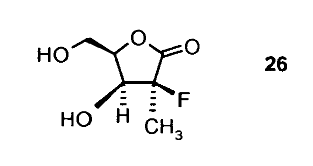

As a rule the acidic treatment in step c) is performed with an acid selected from the group consisting of acetic acid, sulfuric acid, hydrochloric acid, methanesulfonic acid or trifluoroacetic acid, preferably with aqueous sulfuric acid.

Conversion of 22 and 24 to the corresponding 25 and 26 in step (a) may also be carried out using an acid as catalyst in a mixed solution of an organic solvent and water at an elevated temperature. Suitable conditions include acetic acid/water/95°C, Amberlyst 15/acetonitrile/water/82°C, sulfuric acid/ethanol/water/80°C and hydrochloric acid/1-propanol/water/88°C.

The mixture of the compounds 25 and 26 can be isolated usually by neutralizing the reaction mixture with a suitable base, extraction with a suitable organic solvent and subsequent removal of the solvent.

### Step d)

Step d) requires acylating a mixture 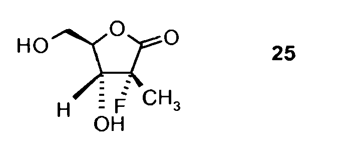 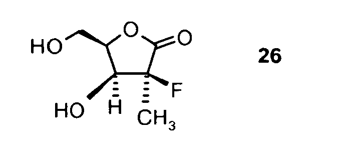 with an acyl halogenide of the formula RCOX wherein R is as above and X is a halogen or with an acyl anhydride RC(O)O(O)CR wherein R is as above in the presence of a base to form a mixture of the compounds 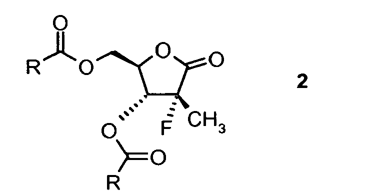 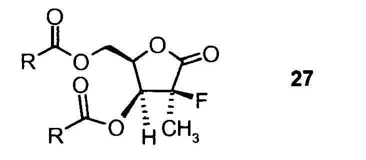 wherein R is as above.

Suitable bases are those which do not react with the acyl halogenide and the acyl anhydride reactant. Non-limiting illustrative examples are pyridine, or tertiary amines such as triethylamine, N,N'-diisopropylamine (DIPEA), 4-dimethylaminopyridine (DMAP). Preferably a tertiary amine and more preferably triethylamine is used as base for the acylation.

Preferred acylation agents are the acyl halogenides RCOX with the R and X having the meaning as above. Most preferred acylating agent is benzoyl chloride.

In a preferred embodiment of the acylation step a catalytic amount of 4-dimethylaminopyridine can be added.

Usually the acylation takes place in a suitable solvent such as in acetonitrile, dimethylformamide (DMF), pyridine, and the like.

As a rule the mixture of the compounds 2 and 27 will not be isolated from the reaction mixture but readily be subjected to the recrystallization in step e)

### Step e)

Step e) requires recyrystallizing a mixture of the compounds 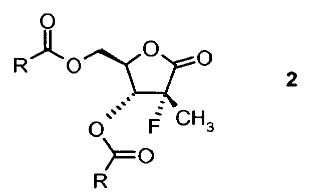 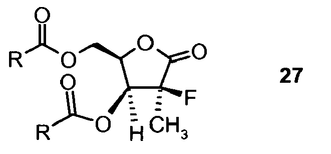 wherein R is as above in an organic solvent and isolating the compound of formula 2.

Usually a water soluble organic solvent is that substantially separates compound 2 in the crystalline form from compound 27. Non-limiting illustrative examples may be selected from the group consisting of methanol, ethanol, *n-*propanol, and isopropanol. A preferred solvent is isopropanol.

In another specific embodiment the present invention provides a method for preparing a compound of the formula: 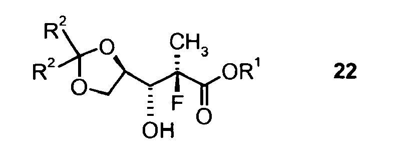 which comprises recrystallizing a mixture of compounds of the formula 22 and 24: 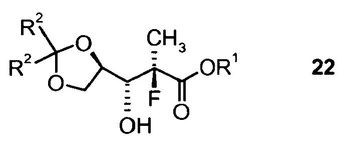 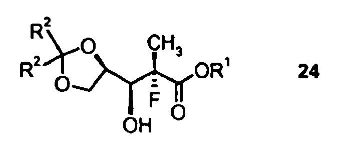 wherein R¹ and R² are as above with an organic solvent.

Usually the mixture of compounds 22 and 24 is dissolved in a suitable solvent such as in tert-butyl methyl ether. Compound 22 can then be precipitated in an apolar solvent, preferably in hexane.

Yet, in another embodiment of the invention a process for the preparation of a compound of formula 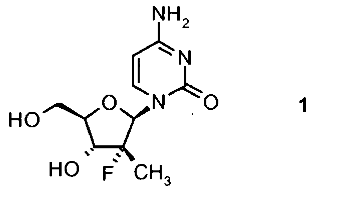 is provided which comprises one or more of the steps as described above.

The following examples shall illustrate the invention without limiting it.

### Examples

### Example 1

Reaction of ethyl 2-fluoropropionate (20, R₁=ethyl) with D-glyceraldehyde, 1,2-acetonide (21, R₂=methyl)

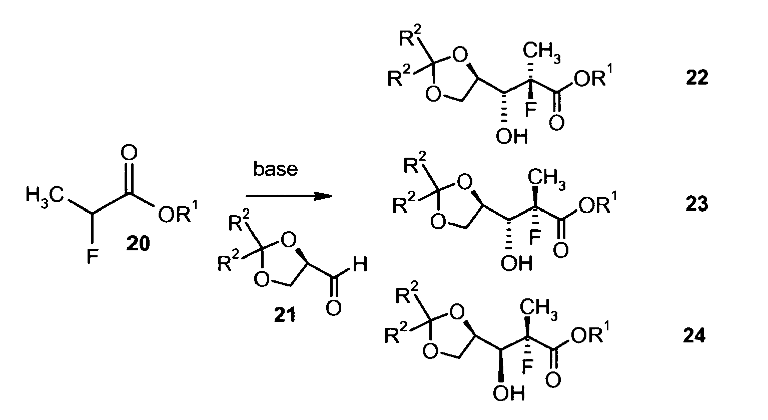

A dry, clean, 2L, 4-neck round bottom flask, equipped with a mechanic stirrer, a thermo couple, a nitrogen inlet, and an addition funnel, was charged with 266 g of anhydrous tetrahydrofuran (THF) and 38.1 g of diisopropylamine. The mixture was stirred and cooled to <-75°C. To the solution was slowly charged 173 g of 1.6 M MeLi solution in ethyl ether while maintaining the batch temperature below -55°C. After the addition the mixture was stirred at approximately -75°C. for 40 minutes. To this mixture was then slowly added 45.2 g of ethyl 2-fluoropropionate 20 while maintaining the batch temperature below -74°C. The mixture was stirred at -76°C. for 50 minutes and a solution of 35 g freshly distilled D-glyceraldehyde, 1,2-acetonide 21 in 178 g of anhydrous THF was slowly added while maintaining batch temperature below -74°C. After the addition the mixture was stirred for approximately 20 minutes. To it was added 300 g of 20% NH₄Cl solution. The mixture was slowly warmed to ambient temperature and transferred to a separatory funnel. The aqueous phase was separated and extracted with 2X132 g =264 g of dichloromethane. The organic phases were combined, dried over MgSO₄, filtered, and concentrated to give 58 g crude aldol product as thick oil. A gas chromatogram showed the oil contained 12.2% of 24, 43.4% of 22, and 35.2% of 23.

### Example 2

Reaction of ethyl 2-fluoropropionate (20, R₁=ethyl) with D-glyceraldehyde, 1,2-pentanonide (21, R₂=ethyl)

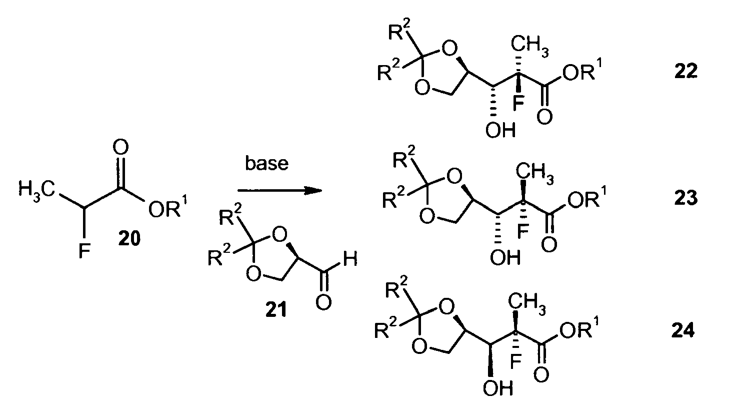

A dry, clean, 4-neck round bottom flask, equipped with a mechanic stirrer, a thermo couple, a nitrogen inlet, and an addition funnel, was charged with 20 mL of anhydrous THF and 1.8 g of diisopropylamine. The mixture was stirred and cooled to <-75°C. To the solution was slowly charged 11 mL of 1.6 M MeLi solution in ethyl ether while maintaining the batch temperature below -55°C. After the addition the mixture was stirred at approximately -75°C. for 30 minutes. To this mixture was then slowly added 2.1 g of ethyl 2-fluoropropionate 20 while maintaining the batch temperature below -74°C. The mixture was stirred at -76 °C for 30 minutes and a solution of 2 g freshly distilled D-glyceraldehyde, 1,2-pentanonide 21 in 10 mL of anhydrous THF was slowly added while maintaining batch temperature below -74 °C. After the addition the mixture was stirred for approximately 20 minutes. To it was added 20 mL of 20% NH₄Cl solution. The mixture was slowly warmed to ambient temperature and transferred to a separatory funnel. The aqueous phase was separated and extracted with 2X10 mL = 20 mL of dichloromethane. The organic phases were combined, dried over MgSO₄, filtered, and concentrated to give 3 g crude aldol product as thick oil. A gas chromatogram showed the oil contained 8.2% of 24, 36.5% of 22, and 30.8% of 23.

### Example 3

Reaction of *n-*butyl 2-fluoropropionate (20, R₁=*n-*butyl) with D-glyceraldehyde, 1,2-acetonide (21, R₂=methyl)

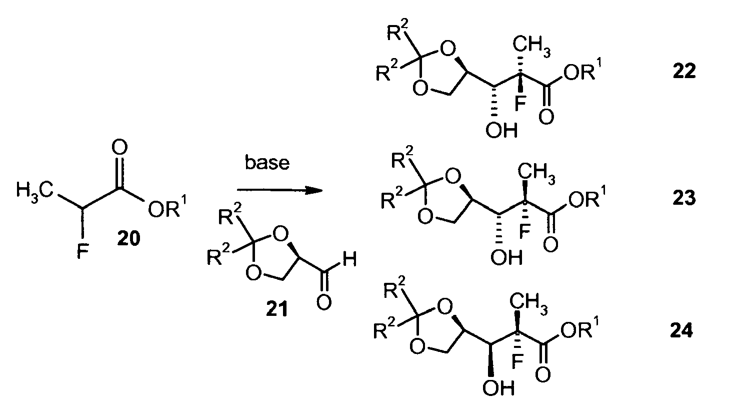

A dry, clean, 4-neck round bottom flask, equipped with a mechanic stirrer, a thermo couple, a nitrogen inlet, and an addition funnel, was charged with 100 mL of anhydrous toluene and 10 g of diisopropylamine. The mixture was stirred and cooled to <-75°C. To the solution was slowly charged 54 mL of 1.6 M MeLi solution in ethyl ether while maintaining the batch temperature below -55°C. After the addition the mixture was stirred at approximately -75°C for 30 minutes. To this mixture was then slowly added 11 g of n-butyl 2-fluoropropionate 20 while maintaining the batch temperature below -70°C. The mixture was stirred at -76°C for 30 minutes and a solution of 8 g freshly distilled D-glyceraldehyde, 1,2-acetonide 21 in 50 mL of anhydrous toluene was slowly added while maintaining batch temperature below -74°C. After the addition the mixture was stirred for approximately 1 hour. The mixture was added to 30 mL of 30% citric acid solution. The mixture was slowly warmed to ambient temperature and transferred to a separatory funnel. The aqueous phase was separated and extracted with 2X20 mL = 40 mL of ethyl acetate. The organic phases were combined, washed with brine, and concentrated to give 11 g crude aldol product as thick oil. A gas chromatogram showed the oil contained 5.5% of 24, 55% of 22, and 39% of 23.

### Example 4

Enzymatic Treatment of a Mixture of 22, 23, and 24 (R₁=ethyl, R₂= methyl)

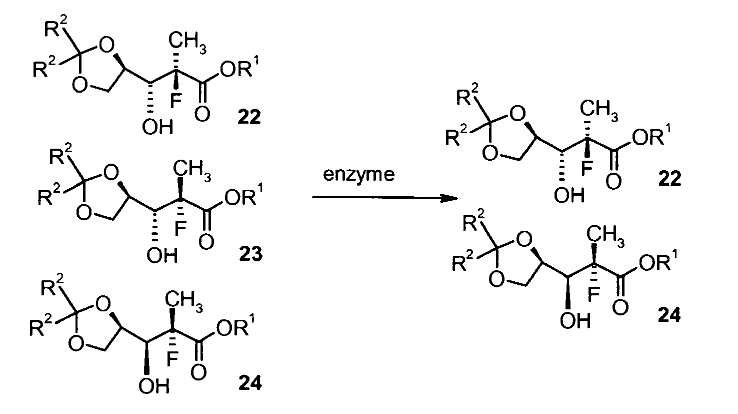

A dry, clean, 1L, 4-neck round bottom flask, equipped with a mechanic stirrer, a thermo couple, a pH probe, and a base dosing pump inlet, was charged with 360 g of buffer which consisted of 10% D-sorbitol, 3mM potassium phosphate, and 38.5 g of crude aldol product (22, 23 and 24). The mixture was stirred at 43°C. (batch temperature) and the pH of the mixture was adjusted to ~7.5 by adding 12% H₂SO₄ solution. To the mixture was added 4 g of CALB solution. The mixture was stirred at the temperature for 22 hours while the pH was maintained at 7.5 by the addition of 1.0 N NaOH solution via a pH pump. The mixture was cooled to ambient temperature, transferred to a separatory funnel, and extracted with 3X100 mL = 300 g of dichloromethane. The organic solution was stirred with 75 g of anhydrous MgSO₄ for 1 hour. The solid was filtered and the filtrate was concentrated to dryness to give 18 g crude mixture of 22 and 24 as thick oil that slowly became a semi-solid.

### Example 5.

Preparation of 2 (R=Ph) from a mixture of 22 and 24 (R₁=ethyl, R₂=methyl)

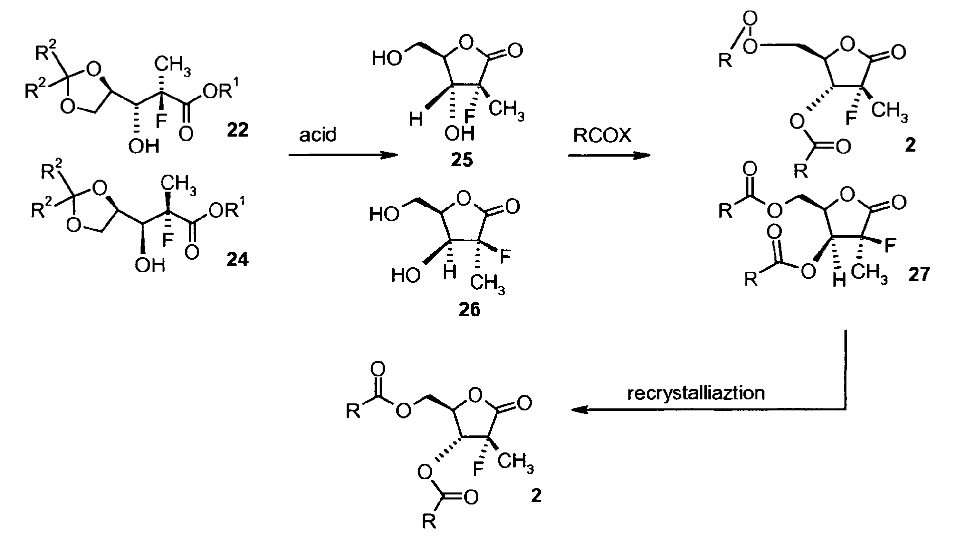

A mixture of 3.0 g of crude mixture of 22 and 24 from Example 4 and 20 g of 2B alcohol and 6 g of 12% sulfuric acid was refluxed at 78°C for 5 hours. The mixture was cooled to ambient temperature and 1 g of triethylamine was added to neutralize the acid. The mixture was concentrated to dryness. The residue was mixed with 20 g of toluene and the mixture was again concentrated to dryness. The residue was dissolved in 15 g of acetonitrile. To the solution was added a catalytic amount of 4-dimethylaminopyridine (DMAP) and 5.2 g of benzoyl chloride. To this mixture was slowly added 4.1 g of triethylamine while maintaining the batch temperature at <40 °C. After the addition the mixture was stirred for 1 hour. The mixture was diluted with 36 g of ethyl acetate and was cooled to 0°C, 25 g of water was added. The mixture was transferred to a separatory funnel and the aqueous phase was separated and extracted again with 20 g of ethyl acetate. The combined organic solution was washed with 20 g of saturated NaHCO₃ solution, dried over MgSO₄, filtered, and concentrated to give a crude oil. The oil was mixed with 27 g of 2-propanol. The mixture was heated to ~60°C to become a clear solution. The mixture was then slowly cooled to 10°C and held for 1 hour. The solid was filtered and the wet cake was washed with 2-propanol and dried under vacuum at 50°C overnight to give 2.0 g of 2 (R=Ph).

### Example 6

Isolation of Pure 22 (R₁=ethyl, R₂=methyl) after Enzymatic Hydrolysis

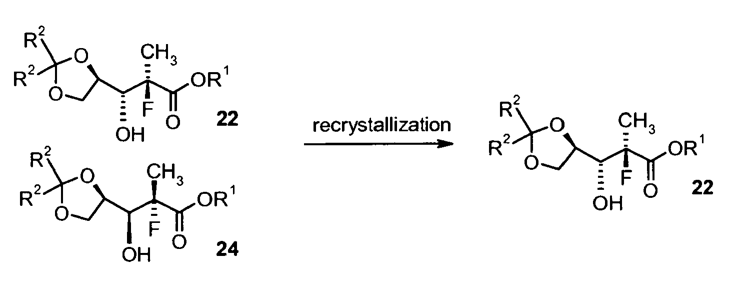

A flask was charged with 3 g of a mixture of 22 and 24 (R₁=ethyl, R₂=methyl) from Example 4 and 3ml of tert-butyl methyl ether (TBME). The mixture was stirred until a clear solution was formed. To this solution was slowly added 10 mL of hexanes. The resulted suspension was stirred at ambient temperature for 2 hours. The solid was isolated and washed with 4 mL of hexane and dried under vacuum at 30°C overnight to give 1.3 g of pure 22 (R₁=ethyl, R₂=methyl).

## Claims

1. Process for the preparation of a compound of the formula: 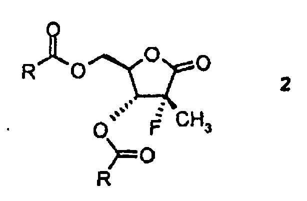 wherein R independently is C₁₋₆-alkyl, optionally substituted phenyl-C₁₋₆-alkyl or optionally substituted phenyl
which comprises one or more of the steps:
a) reacting a compound of the formula 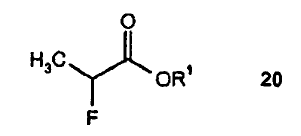 wherein R¹ is C₁₋₄-alkyl,
with a base and a compound of formula 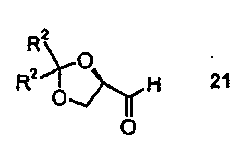 wherein R² is C₁₋₄-alkyl,
to form a mixture of the compounds 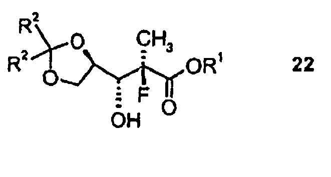 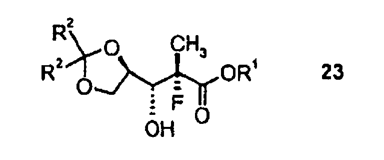 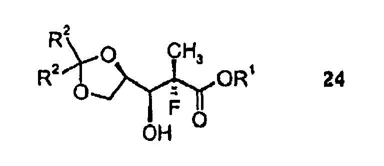 wherein R¹ and R² are as above;
b) reacting a mixture of the compounds 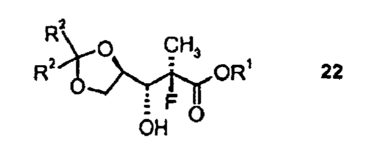 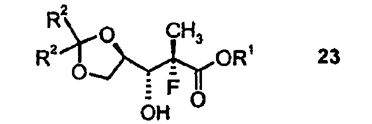 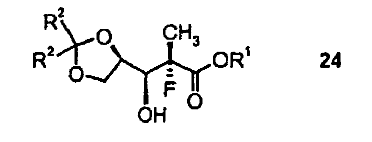 wherein R¹ and R² are as above,
with an enzyme capable to hydrolyze the compound of formula 23 to the corresponding carboxylic acid and isolating a mixture of the compounds 22 and 24;
c) transforming a mixture of the compounds 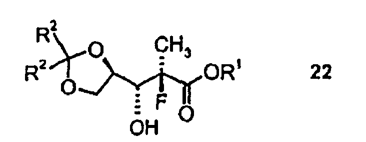 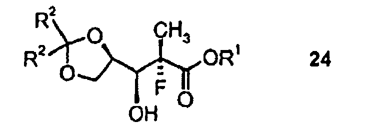 wherein R¹ and R² are as above, by acidic treatment into a mixture of the compounds 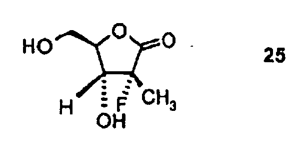 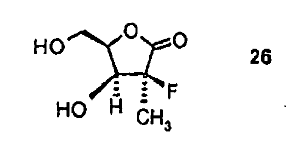
d) acylating a mixture of the compounds 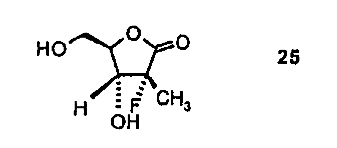 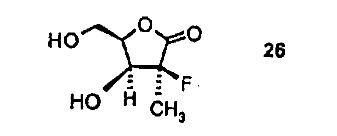 with an acyl halogenide of the formula
RCOX
wherein R is as above and X is a halogen or with an acyl anhydride
RC(O)O(O)CR
wherein R is as above in the presence of a base
to form a mixture of the compounds 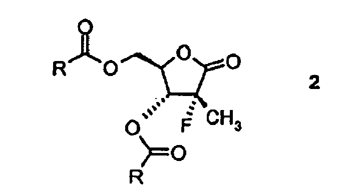 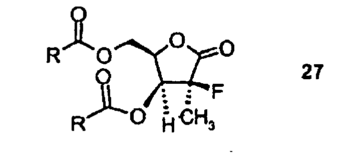 wherein R is as above;
e) recyrystallizing a mixture of the compounds 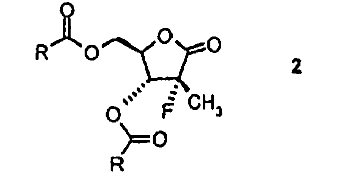 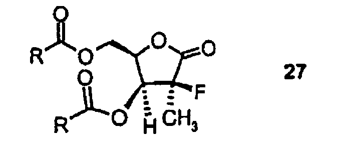 wherein R is as above in an organic solvent and isolating the compound of formula 2.

2. Process according to claim 1 wherein R is phenyl, R₁ is methyl, ethyl or n-butyl, R₂ is methyl or ethyl and X is chloro.

3. Process according to claim 1, **characterized in that** the base used for the reaction step a) is selected from the group consisting of lithium diisopropylamide, lithium 2, 2, 6, 6-tetramethylpiperidine, and lithium hexamethyldisilazide.

4. Process according to claim 1, **characterized in that** the enzyme used in step (b) is a Candida Antarctica Lipase 2 (CALB).

5. Process according to claims 1 or 4, **characterized in that** the enzymatic hydrolysis in step b) is performed in an aqueous buffer medium at a pH between 7.0 and 7.5 and a temperature between 20°C and 45°C.

6. Process according to claims 1, 4 or 5, **characterized in that** in step b) the mixture of the compounds 22 and 24 is recovered by extraction of the reaction mixture with an organic solvent.

7. Process according to claim 1, **characterized in that** the acidic treatment in step c) is performed with an acid selected from the group consisting of acetic acid, sulfuric acid, hydrochloric acid, methanesulfonic acid or trifluoroacetic acid.

8. Process according to claim 1 or 7, **characterized in that** the base used for the acylation in step d) is a tertiary amine.

9. Process according to claims 1 or 8, **characterized in that** the acylation in step d) is performed with benzoylchloride.

10. Process according to claim 1, **characterized in that** the recrystallization in step e) is performed with an aliphatic alcohol selected from methanol, ethanol, n-propanol and isopropanol.

11. Process according to claim 1 or 10, **characterized in that** isopropanol is used.

## Patentansprüche

1. Verfahren zur Herstellung einer Verbindung der Formel: 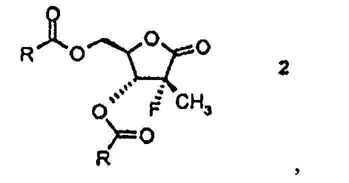 wobei R unabhängig C₁₋₆-Alkyl, gegebenenfalls substituiertes Phenyl-C₁₋₆-alkyl oder gegebenenfalls substituiertes Phenyl ist,
welches einen oder mehrere der Schritte:
a) Umsetzen einer Verbindung der Formel 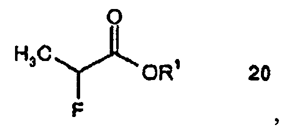 wobei R¹ C₁₋₄-Alkyl ist,
mit einer Base und einer Verbindung der Formel 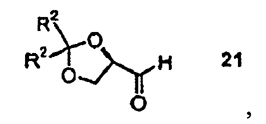 wobei R² C₁₋₄-Alkyl ist,
unter Bildung eines Gemisches der Verbindungen 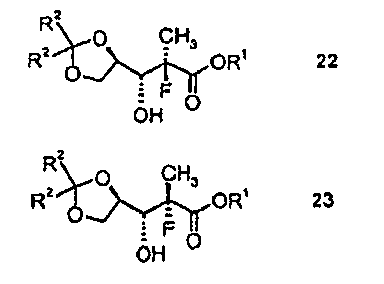 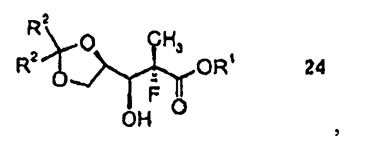 wobei R¹ und R² wie oben sind;
b) Umsetzen eines Gemisches der Verbindungen 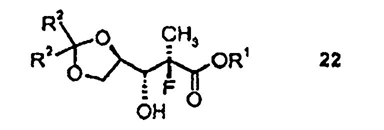 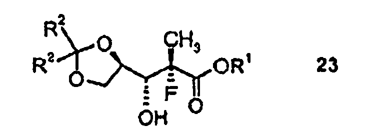 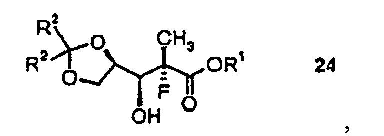 wobei R¹ und R² wie oben sind,
mit einem Enzym, das die Verbindung der Formel 23 zu der entsprechenden Carbonsäure hydrolysieren kann, und Isolieren eines Gemisches der Verbindungen 22 und 24;
c) Umwandeln eines Gemisches der Verbindungen 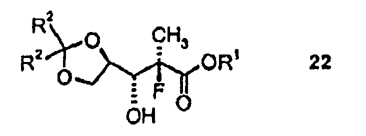 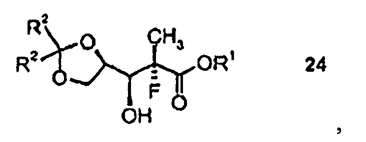 wobei R¹ und R² wie oben sind, durch Säurebehandlung in ein Gemisch der Verbindungen 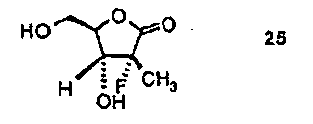 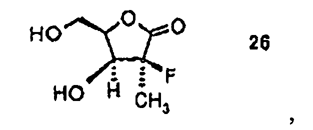
d) Acylieren eines Gemisches der Verbindungen 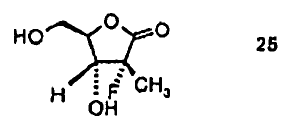 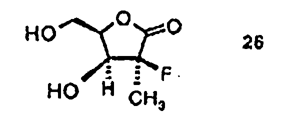 mit einem Acylhalogenid der Formel
RCOX,
wobei R wie oben ist und X Halogen ist, oder mit einem Acylanhydrid
RC(O)O(O)CR,
wobei R wie oben ist, in Gegenwart einer Base
unter Bildung eines Gemisches der Verbindungen 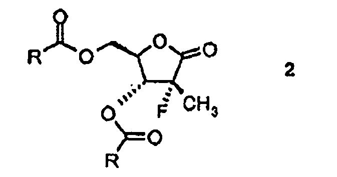 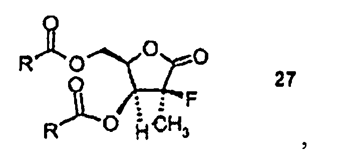 wobei R wie oben ist;
e) Umkristallisieren eines Gemisches der Verbindungen 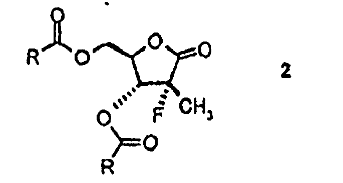 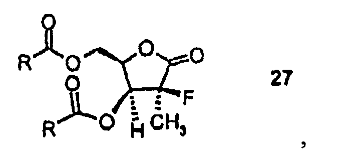
wobei R wie oben ist, in einem organischen Lösungsmittel und Isolieren der Verbindung der Formel 2 umfasst.

2. Verfahren nach Anspruch 1, wobei R Phenyl ist, R₁ Methyl, Ethyl oder n-Butyl ist, R₂ Methyl oder Ethyl ist und X Chlor ist.

3. Verfahren nach Anspruch 1, **dadurch gekennzeichnet, dass** die für die Umsetzung in Schritt a) verwendete Base aus der Gruppe ausgewählt ist, bestehend aus Lithiumdiisopropylamid, Lithium-2,2,6,6-tetramethylpiperidin und Lithiumhexamethyldisilazid.

4. Verfahren nach Anspruch 1, **dadurch gekennzeichnet, dass** das in Schritt (b) verwendete Enzym eine Candida-Antarctica-Lipase-2 (CALB) ist.

5. Verfahren nach den Ansprüchen 1 oder 4, **dadurch gekennzeichnet, dass** die enzymatische Hydrolyse in Schritt b) in einem wässerigen Puffermedium bei einem pH zwischen 7,0 und 7,5 und einer Temperatur zwischen 20 °C und 45 °C durchgeführt wird.

6. Verfahren nach den Ansprüchen 1, 4 oder 5, **dadurch gekennzeichnet, dass** in Schritt b) das Gemisch der Verbindungen 22 und 24 durch Extraktion des Reaktionsgemisches mit einem organischen Lösungsmittel gewonnen wird.

7. Verfahren nach Anspruch 1, **dadurch gekennzeichnet, dass** die Säurebehandlung in Schritt c) mit einem Säure, ausgewählt aus der Gruppe, bestehend aus Essigsäure, Schwefelsäure, Salzsäure, Methansulfonsäure oder Trifluoressigsäure, durchgeführt wird.

8. Verfahren nach Anspruch 1 oder 7, **dadurch gekennzeichnet, dass** die für die Acylierung in Schritt d) verwendete Base ein tertiäres Amin ist.

9. Verfahren nach den Ansprüchen 1 oder 8, **dadurch gekennzeichnet, dass** die Acylierung in Schritt d) mit Benzoylchlorid durchgeführt wird.

10. Verfahren nach Anspruch 1, **dadurch gekennzeichnet, dass** die Umkristallisierung in Schritt e) mit einem aliphatischen Alkohol, ausgewählt aus Methanol, Ethanol, n-Propanol und Isopropanol, durchgeführt wird.

11. Verfahren nach Anspruch 1 oder 10, **dadurch gekennzeichnet, dass** Isopropanol verwendet wird.

## Revendications

1. Procédé de préparation d'un composé de formule: 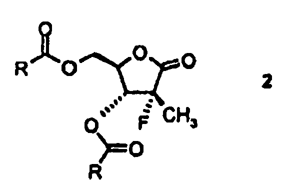 dans laquelle R est indépendamment alkyle en C₁-C₆, phényl-(alkyle en C₁-C₆) éventuellement substitué ou phényle éventuellement substitué,
qui comprend une ou plusieurs des étapes:
a) réaction d'un composé de formule 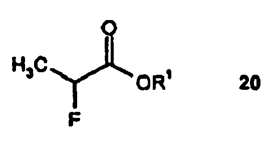 où R¹ est alkyle en C₁-C₄,
avec une base et un composé de formule 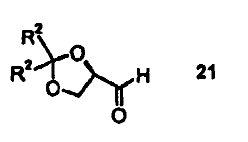 où R² est alkyle en C₁-C₄,
pour former un mélange des composés 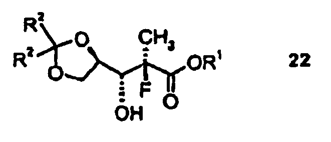 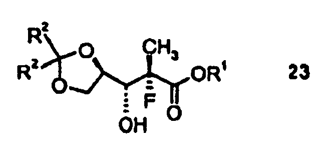 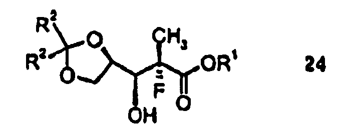 où R¹ et R² sont comme ci-dessus;
b) réaction d'un mélange des composés 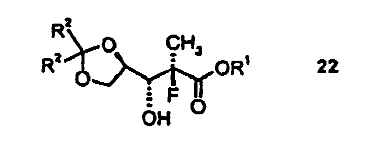 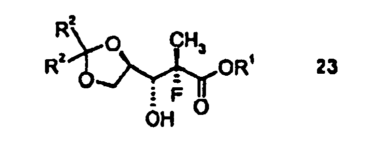 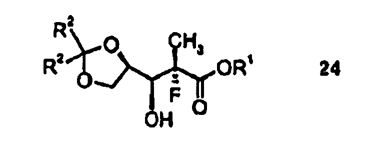 où R¹ et R² sont comme ci-dessus,
avec une enzyme capable d'hydrolyser le composé de formule 23 en l'acide carboxylique correspondant et isolement d'un mélange des composés 22 et 24;
c) transformation d'un mélange des composés 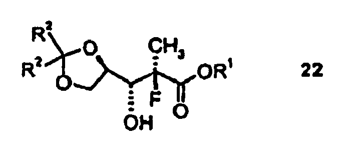 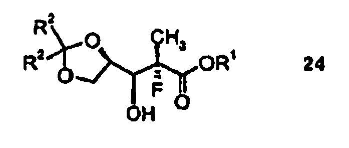 où R¹ et R² sont comme ci-dessus, par traitement acide, en un mélange des composés 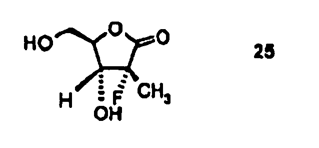 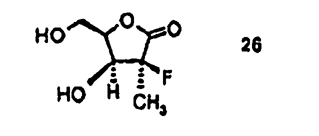
d) acylation d'un mélange des composés 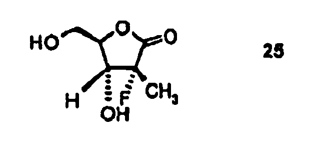 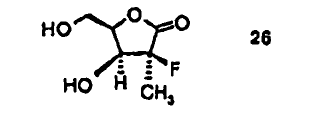 avec un halogénure d'acyle de formule
RCOX
où R est comme ci-dessus et X est un halogène, ou avec un anhydride d'acyle
RC(O)O(O)CR
où R est comme ci-dessus, en présence d'une base, pour former un mélange des composés 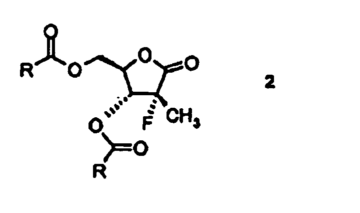 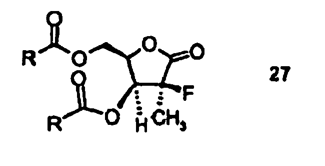 où R est comme ci-dessus;
e) recristallisation d'un mélange des composés 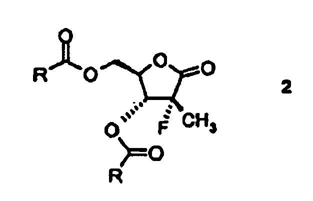 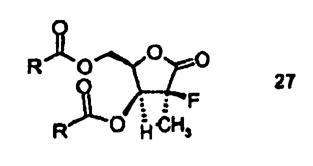 où R est comme ci-dessus, dans un solvant organique, et isolement du composé de formule 2.

2. Procédé selon la revendication 1, où R est phényle, R¹ est méthyle, éthyle ou n-butyle, R² est méthyle ou éthyle et X est chloro.

3. Procédé selon la revendication 1, **caractérisé en ce que** la base utilisée pour l'étape de réaction a) est choisie dans le groupe constitué par le diisopropylamidure de lithium, la 2,2,6,6-tétraméthylpipéridine-lithium, et l'hexaméthyldisilazide de lithium.

4. Procédé selon la revendication 1, **caractérisé en ce que** l'enzyme utilisée dans l'étape (b) est une lipase 2 de *Candida Antarctica* (CALB).

5. Procédé selon les revendications 1 ou 4, **caractérisé en ce que** l'hydrolyse enzymatique dans l'étape b) s'effectue dans un milieu de tampon aqueux à un pH entre 7,0 et 7,5 et une température entre 20°C et 45°C.

6. Procédé selon les revendications 1, 4 ou 5, **caractérisé en ce que**, dans l'étape b), on récupère le mélange des composés 22 et 24 par extraction du mélange réactionnel avec un solvant organique.

7. Procédé selon la revendication 1, **caractérisé en ce que** le traitement acide dans l'étape c) s'effectue avec un acide choisi dans le groupe constitué par l'acide acétique, l'acide sulfurique, l'acide chlorhydrique, l'acide méthanesulfonique ou l'acide trifluoroacétique.

8. Procédé selon la revendication 1 ou 7, **caractérisé en ce que** la base utilisée pour l'acylation dans l'étape d) est une amine tertiaire.

9. Procédé selon les revendications 1 ou 8, **caractérisé en ce que** l'acylation dans l'étape d) s'effectue avec du chlorure de benzoyle.

10. Procédé selon la revendication 1, **caractérisé en ce que** la recristallisation dans l'étape e) s'effectue avec un alcool aliphatique choisi parmi le méthanol, l'éthanol, le n-propanol et l'isopropanol.

11. Procédé selon la revendication 1 ou 10, **caractérisé en ce que** l'on utilise de l'isopropanol.
